# EUROPEAN PATENT SPECIFICATION

(11) **EP 0 541 249 B1**
(45) Date of publication and mention of the grant of the patent: **03.07.1996**
(21) Application number: 92309188.8
(22) Date of filing: 08.10.1992
(51) Int. Cl.: A61F 9/007

(54) **Ultrasonic handpiece**
Handstück für Ultraschallvorrichtung
Outil ultrasonique à préhension manuelle

(30) Priority: 08.10.1991 US 773132
(43) Date of publication of application: 12.05.1993
(60) Divisional application: 95203676.2
(73) Proprietor: SURGICAL DESIGN CORPORATION, Long Island City, New York 11101 (US)
(72) Inventor: Strukel, Igor, New York NY 10009 (US)
(74) Representative: Gallafent, Richard John

(56) References cited:
- WO-A-87/00422
- WO-A-91/07917
- GB-A- 2 116 045
- US-A- 4 169 984
- US-A- 4 808 154

## Description

Various types of ultrasonic handpieces are used for surgical application, for example, ophthalmological surgery for removing cataracts or other tissue. Typically, such handpieces use some type of a vibrating transducer of the magnetostrictive or piezoelectric type which converts electrical energy to mechanical vibration.

Such types of ultrasonic handpieces are also capable of supplying irrigation fluid to the operating site in which the transducer is to be used, and also to produce suction for removing the material which has been emulsified by the ultrasonic transducer. It is also known that, in such ultrasonic transducers, the irrigation fluid and the suction are supplied through tubes associated with the transducer and partially or totally within the housing which houses the transducer. Usually, in the case of a magnetostrictive type of transducer, the tubes are laid along and outside the stack of laminations forming the vibrating structure or at least partially outside of the housing. It is desirable to reduce the overall size of the housing so that the transducer becomes easier for the user to hold and manipulate.

It is also often desirable to increase the operating power output of such transducers. In magnetostrictive type transducers, this can be done by increasing the size of the lamination stack and/or the coil wire size to provide additional current. Neither approach offers desirable solutions since they increase the transducer size and/or its heat output.

In addition, it is desired to improve the emulsification action and to confine it more closely to or within the work site.

WO-A-9107917 describes an ultrasonic surgical instrument in the form of a handpiece including an ultrasonic transducer and a transition body for transmitting the ultrasonic vibrations from the transducer to a detachable tip member. Means for supplying an irrigation liquid include a passage through the transition body, which feeds liquid to the exterior of the tip. The working end of the tip is surrounded by an external sleeve, the irrigation liquid passing between the sleeve and the tip member.

It has been found that the irrigation fluid to be supplied to the operation site interacts with the vibrating structure to produce a cavitation effect, which reduces the transducer power output. In accordance with the present invention, an elastic membrane covers the vibrating structure and isolates it from the irrigation fluid. This permits the structure to vibrate more freely, with a consequential increase in the transducer power stroke. No increase of either the lamination stack size or coil current is needed.

The membrane provided between the infusion fluid and the vibrating body which supplies power to vibrate the instrument tip reduces the formation of air bubbles arising from cavitation and thereby increases the power stroke of the tip.

The handpiece tip may have an enlarged hollow front end forming a cavity into which the material to be emulsified is drawn. A major part of the ultrasonic energy is radiated from an angled portion within the cavity and the material is emulsified there.

The present invention will become more apparent upon reference to the following description of embodiments thereof, read in conjunction with the annexed drawings in which:
Figures 1a and 1b taken together show a longitudinal view of the ultrasonic handpiece of the present invention in axial cross-section;
Figure 2 is a cross-sectional view of a portion of the handpiece looking along line 2-2 of Figure 1a;
Figure 3 is a cross-sectional view of the hand piece taken along line 3-3 of Figure 1b;
Figure 4 is an enlarged axial cross-section of the end of the work tip of the handpiece shown in Figure 1, and
Figure 5A and 5b are views of an alternative form of sleeve for the handpiece tip.

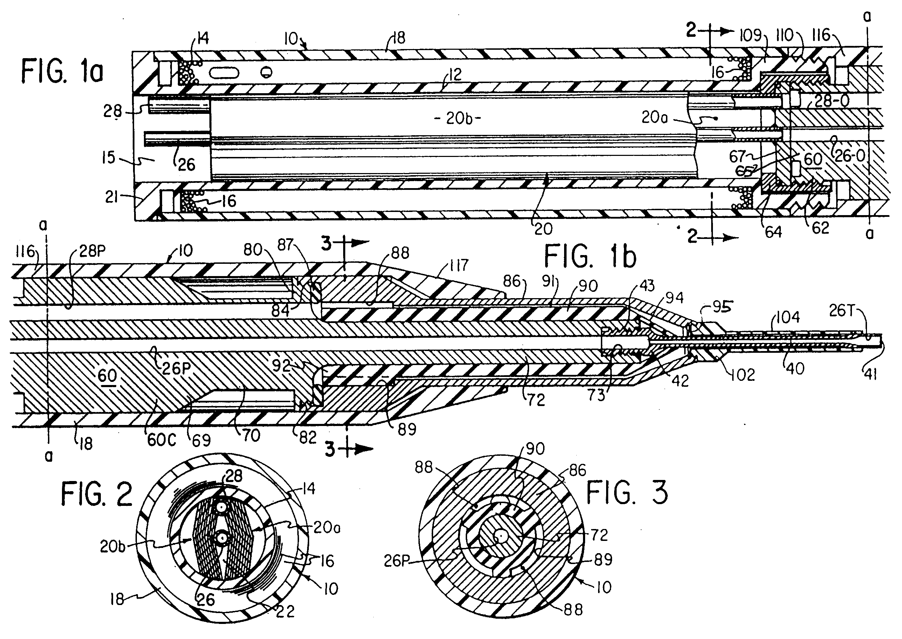

Referring to Figures 1a and 1b, which are to be joined together at lines a-a, and Figures 2-4, the ultrasonic handpiece 10 includes an elongated, generally-cylindrical, housing 12 of a suitable material, for example, TECHTRON (Registered Trade Mark) plastics, having an annular recess 14 along part of its length. Wound around the housing, along the length of recess 14, is a coil 16 of wire. The wire can be either circular in cross section or else, if desired, flattened so that a larger current-carrying surface can be provided. The ends of the wire coil (not shown) are connected by any suitable means to a source of voltage of the proper magnitude.

The recess 14 and coil 16 are covered by a sleeve 18, also of a suitable plastics material, and there is an end cap 21 which closes the housing structure.

In the central hollow 15 of the housing 12 is a stack of laminations 20. The laminations are elongated and extend for a substantial portion of the length of the housing. The laminations are preferably of nickel or other suitable material. As shown in Figure 2, the laminations in stack 20 are split into two groups 20a and 20b. The individual laminations of each of the groups 20a, 20b are bent into a general V-shape and are interleaved with each other while being kept in metallic contact. The two groups of laminations 20a, 20b are then placed adjacent to each other with the housing.

As seen in Figure 2, using the split lamination stack 20a, 20b provides an open central area or cavity 22 through which a tube 26 extends. A second tube 28 extends along the upper (as viewed) part of the stack and is enclosed only partly by the ends of the laminations.

Tube 26 is a suction tube, i.e. its left-hand end (as viewed) is connected to a suitable suction source (not shown) which serves for the removal of material emulsified by the handpiece. Tube 28 is connected at the same end of the housing to a source of suitable liquid (not shown) and serves as an irrigation channel for liquid to be conveyed to the operating site.

With the arrangement as shown, both of the tubes 26 and 28 are contained substantially within the open central area of the split lamination stack rather than outside the stack, as in known handpieces. This permits the housing to have a reduced diameter, which is advantageous since it makes the handpiece easier to handle. It also serves to use the irrigation and suction liquids to cool the laminations to prevent them from overheating.

The handpiece also has an elongated hollow tip 40 which is vibrated and which performs the actual emulsification of the material at the operating site. Tip 40 has an open end 41 which is somewhat enlarged in the shape of a scoop or cup relative to the remainder of the tip length. The end plane of the tip can be oblique or normal to the tip axis.

The tip 40 is provided with the vibratory energy produced by the lamination stack 20 through a transition body 60, which is preferably of titanium. The transition body has a first section 62 which is adjacent the end of the lamination stack 20. The section 62 is coupled to the stack 20 through a collar 64 having internal screw-threads which screw onto the end 65 of the transition body against a plate 67, of a corrosion-resistant alloy of nickel and copper, or similar material, to which the ends of the laminations of stack 20 are attached brazed or otherwise. By this arrangement, there is a secure mechanical coupling of the end of the lamination stack to the transition body 62, so that the vibratory energy produced by the stack, as it is excited by the electrical current, is efficiently transmitted to the transition body 60.

The plate 67 has openings 26-0 and 28-0 into which the ends of the tubes 26 and 28 are inserted and are attached thereto in a fluid-tight manner, for example, by welding or brazing, if the tubes 26, 28 are of metal. If the tubes are of plastics, a suitable adhesive is used.

The transition body 60 continues toward the tip 40 with a central section 60-C, which is its largest diameter portion. The body tapers at 69 to a section of reduced diameter 70, from which extends an elongated cylindrical end section 72 of further reduced diameter, to which tip 40 is attached. The free end 43 of the end section 72 has internal screw-threads 73. The connection of the tip 40 to the transition body is by the inner end of the tip having an enlarged neck 42 with external screw-threads which engage with threads 73 on end 72 of the transition body. In this manner, the vibratory energy produced by the lamination stack 20 is coupled through the transition body 60 to the end 41 of the tip 40.

A passage 26P extends the length of the transducer body to the tip end, and a passage 28P to the end of the transition body central section 70.

The reduced-diameter portion 70 of the transition body has a flange 80 with external screw-threads 82. To this is coupled a metal shell 86, preferably of titanium, which surrounds the elongated end 72 of the transition body and has screw-threads 84 at one end which mate with the threads 82 on the transition body flange 80. An O-ring seal 87 is provided between the shell 86 and the transition body flange 80. The fluid which flows in the aspiration tube and passage 28, 28P flows from the end of passage 28P into a recess 88, from which it flows through passage 91 between the shell 86 and the outside of an elastomeric body 90, which is described below.

The body 90, preferably of silicone plastics, or other suitable elastomeric material, encircles the elongated section 72 of the transition body. The body 90 has one end 92 of enlarged diameter which fits within a corresponding internal recess 89 of the metal shell 86. As the shell 86 is screwed on to the threads 84 of flange 80, the body 90 is secured in place. The outer end 94 of the body 90 is tapered and thinned to extend over the junction between the transition section 72 and the tip 40, adjacent neck 42.

The outer end of the shell 86 has internal screw-threads 95 into which the screw-threaded enlarged nose 102 of an elongated tip sleeve 104 is fastened. The tip sleeve 104, of POLYSULFONE or other suitable plastics material, extends for a suitable portion of the length of the tip 40, terminating at its flared end 41. As shown, the inner diameter of the sleeve 104 is only slightly larger than the outer diameter of the tip end 41. Thus, there is no large step between the tip and the sleeve, which would make it more difficult to see the exact placement of the end of the tip at the operating site.

The purpose of the sleeve 104 is to provide a passage and outlet for the irrigation liquid, which flows from the end of passage 28P, into the space between the shell 86 and the body 90, between the nose 102 of the tip sleeve 104 and the tip body 40, and out of the end of the sleeve 104. Thus the irrigation liquid leaves the handpiece at the operating site. If desired, the sleeve 104 can have one or more openings on its periphery through which the liquid can escape.

The body 90 performs an important function, in that it isolates the irrigation liquid from contact with the outer end 72 of transition body. If there were such contact there would be a cavitation effect in the irrigation fluid because of the vibration of the end portion 72. This would dissipate power and thereby reduce the amount of power present at the tip end 41. By providing the body 90, the power stroke at the tip end 41 increases, without any change in the other structure of the handpiece, such as the lamination stack 20 on coil 16.

Tube 26 applies suction to the tip 40 at its end 41. This draws the material to be emulsified into the enlarged hollow end, which is essentially a cavity 44. The cavity inner wall radiates ultrasonic energy within the cavity, particularly from the internal corners 41a. Thus, the material within the cavity 44 at the tip end is emulsified. The outer rim of the tip end also radiates some energy outwardly but the amount is minor compared to that which is radiated within the cavity. Thus, most of the emulsification occurs within, rather than outside of, the tip.

The emulsified material is conveyed back through the suction passage 46 and the total length of the passage 26-P and the tube 26 and outwardly of the handpiece to a collection chamber (not shown) which is remote from the instrument.

The end of the handpiece housing 12 closest to the tip 40 has a shoulder 109 beyond the adjacent end of housing cover 18. The shoulder is threaded at 110 and an end housing cover 116, which also can be of the same material as housing 12, is connected thereto. The housing end cover 116 extends for part of the length of the transition member about half way along the length of the reduced diameter portion 72. The other end of cover 116 has a tapered end 117 which contacts the shell 86, permitting the person using the handpiece to hold it by the cover 116 and shell 86.

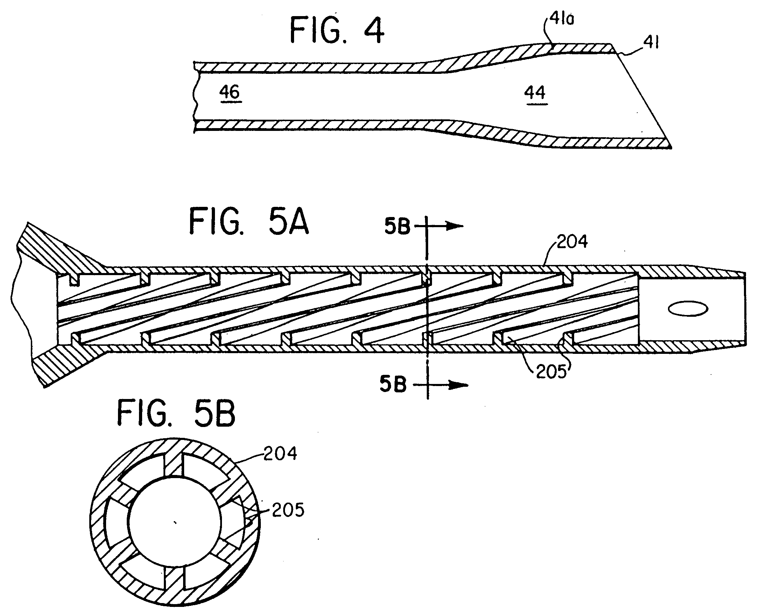

The tip sleeve 104 is made of a relatively-hard plastics material, such as that sold under the trade mark POLYSUFONE. This has an advantage in that it does not absorb ultrasonic energy. In some cases it is preferred that a softer plastics be used. Figures 5 and 5A showing another embodiment of the sleeve 204 for securing the tip. Here the sleeve material 204 is of a softer plastics, such as silicone rubber. It has several multistart internal helicoidal ribs 205 which run along its length. The inner ends of the ribs 205 engage the outer surface of the tip 40 but leave a space along which the irrigant liquid can flow. Since the entire inner surface of the sleeve 204 is not in contact with the tip and since the ribs 205 stiffen the sleeve along its length, the amount of energy that the sleeve absorbs is reduced compared with the sleeve 104.

The present invention provides an ultrasonic handpiece of which the output power is increased without increase in size. The handpiece of the present invention can produce the same power as a handpiece of larger size which does not split the lamination stack to accept the fluid tubes and/or which does not use the an elastomeric body to prevent cavitation. Further, better control of the emulsification and tissue removal is obtained by the shape of the tip end.

While the preferred embodiment of the invention has been described with respect to using a magnetostrictive transducer, it will also operate with other types of transducer, such as piezoelectric.

## Claims

1. A handpiece for directing ultrasonic energy at an object, comprising:
a transducer (16,20) for converting electrical energy into vibrations at an ultrasonic frequency;
a transition body (60) for transmitting the vibrational energy to a detachable tip member (40), the body having in it at least one passage (28P) for conveying an irrigation liquid to the tip member;
and characterised by
a sleeve (90) of elastomeric material embracing the tip end (72) of the transition body (60), and
an external shell (86) spaced outwardly of the sleeve to define therebetween a passage (91) in fluid communication with the irrigation passage (28P).

2. A handpiece as claimed in Claim 1, in which the tip member (40) is hollow, having its interior in fluid communication with one of the conduits (26) in the housing through a passage (26P) in the transition body (60).

3. A handpiece as claimed in Claim 2, in which the transition body has part (72) of its length embraced by a sleeve (90) of elastomeric material, in which the transition body supports a shell (86) encircling the sleeve and forming with it a fluid-flow passage in communication with a conduit (28) in the housing through a passage (28P) in the transition body, whereby for part of its length the transition body is mechanically insulated from the said fluid-flow passage.

4. A handpiece as claimed in Claim 1 or 3, in which one end of the sleeve (90) extends beyond the respective end of the transition body (60) and is adapted to be in fluid-tight contact with an outer surface of the tip member (40) when in position on the handpiece.

5. A handpiece as claimed in any preceding claim, in which the other end of the transition body (60) is detachably connected to a housing (12) for the transducer, the housing including at least one conduit (26,28) for liquid, the or each conduit being secured at one end to a support (67) able to be clamped to one end of the transition body, with the interior of the or each conduit in fluid communication with a respective passage (28P,26P) in the transition body.

## Patentansprüche

1. Handstück zum Richten von Ultraschallenergie auf ein Objekt, das umfaßt:
einen Wandler (16, 20), der elektrische Energie in Schwingungen mit Ultraschallfrequenz umwandelt;
einen Übergangskörper (60), der die Schwingungsenergie auf ein abnehmbares Spitzenelement (40) überträgt, wobei der Körper wenigstens einen Durchlaß (28P) darin zum Transport einer Spülflüssigkeit zu dem Spitzenelement aufweist;
und **gekennzeichnet durch**
eine Hülse (90) aus Elastomermaterial, die das Spitzenende (72) des Übergangskörpers (60) umschließt, und
eine äußere Ummantelung (86), die nach außen von der Hülse beabstandet ist, so daß zwischen ihnen ein Durchlaß (91) gebildet wird, der mit dem Spüldurchlaß (28P) in Fluidverbindung steht.

2. Handstück nach Anspruch 1, wobei das Spitzenelement (40) hohl ist und sein Inneres über einen Durchlaß (26P) in dem Übergangskörper (60) mit einer der Leitungen (26) in dem Gehäuse in Fluidverbindung steht.

3. Handstück nach Anspruch 2, wobei ein Teil (72) der Länge des Übergangskörpers von einer Hülse (90) aus Elastomermaterial umschlossen ist, wobei der Übergangskörper eine Ummantelung (86) trägt, die die Hülse einschließt und mit ihr einen Fluidstromdurchlaß bildet, der über einen Durchlaß (28P) in dem Übergangskörper mit einer Leitung (28) in dem Gehäuse in Verbindung steht, wobei ein Teil der Länge des Übergangskörpers gegenüber dem Fluidstromdurchlaß mechanisch isoliert ist.

4. Handstück nach Anspruch 1 oder 3, wobei sich ein Ende der Hülse (90) über das entsprechende Ende des Übergangskörpers (60) hinaus erstreckt und mit einer Außenfläche des Spitzenelementes (40) in fluiddichtem Kontakt ist, wenn es sich an dem Handstück in Position befindet.

5. Handstück nach einem der vorangehenden Ansprüche, wobei das andere Ende des Übergangskörpers (60) lösbar mit einem Gehäuse (12) für den Wandler verbunden ist, wobei das Gehäuse wenigstens eine Leitung (26, 28) für Flüssigkeit enthält, wobei die oder jede Leitung an einem Ende an einer Halterung (57) befestigt ist, die an einem Ende des Übergangskörpers angeklemmt werden kann, wobei das Innere der oder jeder Leitung mit einem entsprechenden Durchlaß (28P, 26P) in dem Übergangskörper in Fluidverbindung steht.

## Revendications

1. Pièce à main destinée à canaliser de l'énergie ultrasonore vers un objet, comprenant :
un transducteur (16, 20) destiné à convertir de l'énergie électrique en vibrations à une fréquence ultrasonore,
un corps intermédiaire (60) destiné à transmettre l'énergie vibratoire à un élément de pointe amovible (40), le corps comportant en lui-même au moins un passage (28P) destiné à acheminer un liquide d'irrigation vers l'élément de pointe,
et caractérisée par
un manchon (90) de matériau élastomère renfermant l'extrémité de pointe (72) du corps intermédiaire (60), et
une enveloppe externe (86) espacée de l'extérieur du manchon pour définir entre ceux-ci un passage (91) en communication fluidique avec le passage d'irrigation (28P).

2. Pièce à main selon la revendication 1, dans laquelle l'élément de pointe (40) est creux, son intérieur étant en communication fluidique avec l'un des conduits (26) du boîtier par l'intermédiaire d'un passage (26P) ménagé dans le corps intermédiaire (60).

3. Pièce à main selon la revendication 2, dans laquelle le corps intermédiaire comporte une partie (72) de sa longueur enfermée par un manchon (90) de matériau élastomère, dans laquelle le corps intermédiaire supporte une enveloppe (86) entourant le manchon et formant avec celui-ci un passage d'écoulement de fluide en communication avec un conduit (28) du boîtier par l'intermédiaire d'un passage (28P) ménagé dans le corps intermédiaire, d'où il résulte que sur une partie de sa longueur, le corps intermédiaire est isolé mécaniquement dudit passage d'écoulement de fluide.

4. Pièce à main selon la revendication 1 ou la revendication 3, dans laquelle une extrémité du manchon (90) s'étend au-delà de l'extrémité respective du corps intermédiaire (60) et est conçue pour venir en contact étanche aux fluides avec une surface extérieure de l'élément de pointe (40) lorsqu'il est en position dans la pièce à main.

5. Pièce à main selon l'une quelconque des revendications précédentes, dans laquelle l'autre extrémité du corps intermédiaire (60) est reliée de façon amovible à un boîtier (12) destiné au transducteur, le boîtier comprenant au moins un conduit (26, 28) pour un liquide, le conduit ou chaque conduit étant fixé à une extrémité sur un support (67) qui peut être serré contre une extrémité du corps intermédiaire, l'intérieur du conduit ou de chaque conduit étant en communication fluidique avec un passage respectif (28P, 26P) ménagé dans le corps intermédiaire.
